# EUROPEAN PATENT SPECIFICATION

(11) **EP 2 633 828 B1**
(45) Date of publication and mention of the grant of the patent: **07.08.2019**
(21) Application number: 13275028.2
(22) Date of filing: 13.02.2013
(51) Int. Cl.: A61B 17/34

(54) **Introducer assembly**
Einführerbaugruppe
Ensemble introducteur

(30) Priority: 28.02.2012 US 201261604329 P
(43) Date of publication of application: 04.09.2013
(73) Proprietor: Cook Medical Technologies LLC, Bloomington, IN 47404 (US)
(72) Inventor: Ahmed, Mahfuza, 02467 Chestnut Hill (MA); Ryan, Walter N., 27858 Greenville (NC)
(74) Representative: Jehan, Robert

(56) References cited:
- AU-A1- 2010 201 228
- US-A- 5 098 392
- US-A1- 2004 260 246
- US-B1- 6 589 262

## Description

### TECHNICAL FIELD

The technical field of this invention is implantable medical devices, and in particular, a dilator and sheath useful for performing percutaneous nephrostomy procedures.

### BACKGROUND

Minimally-invasive surgery has evolved to a point where procedures that were unimaginable a few years ago are now routinely performed on a daily basis. These procedures may involve a physician operating internally on a patient's body through a relatively small opening. Access to the small opening may be obtained percutaneously by opening a tract into a patient's body and inserting a sheath through the tract. The sheath helps to maintain the patency of the tract and facilitates the insertion, placement and removal of endoluminal devices to the target area.

One particular procedure involving the introduction of a sheath for the performance of minimally-invasive surgery is a percutaneous nephrostomy. Percutaneous nephrostomy is a procedure that can be used to provide percutaneous access to the upper urinary tract by creating an opening between the renal pelvis and the outside of the body. Additionally, percutaneous nephrostomy may be used to remove or dissolve renal calculi, to obtain direct access to the upper urinary tract for various endourologic procedures, to diagnose ureteral obstruction, filling defects, and anomalies via antegrade radiography, to deliver chemotherapeutic agents to the renal collecting system, and to provide prophylaxis after resection for local chemotherapy in patients with tumors of the renal pelvis. Prior to inserting the sheath, generally a small tract is made with a needle through a patient's body and into the renal pelvis. Following creation of the tract, a physician inserts one or more dilation catheters to increase the size of the tract to the kidney and to allow for proper introduction of the sheath. Because axial pressure is required to advance and place the sheath, care must be taken to avoid kinking the sheath too far and perforating the renal pelvis. The procedure also requires multiple steps to provide access to the target area. Furthermore, for obese patients, the procedure provides the possibility that the sheath may get displaced within subcutaneous layers of fat tissue, which may result in difficulties in performing the procedure, and cause the patient additional trauma.

US 6,589,262 B1 discloses an introducer assembly including an introducer sheath, a hub attached about the proximal end of the introducer sheath, the hub having an opening disposed through its center in fluid communication with the lumen of the introducer sheath, and a rotatable fastener disposed on the base and including a dilator, the dilator being coaxially positioned within the lumen of the introducer sheath.

### BRIEF SUMMARY

Claim 1 defines the invention and the dependent claims disclose the preferred embodiments. The present invention seeks to provide an improved introducer assembly. According to an aspect of the present invention, there is provided an introducer assembly as specified in claim 1.

An embodiment of the invention comprises:
an introducer sheath having a proximal end and a lumen disposed therethrough, a hub molded to the proximal end of the introducer sheath, the hub comprising a main body having at least one opening disposed through its center in fluid communication with the introducer sheath and a wire guide receiving member attached about a periphery of the main body;
and a dilator clip releasably attached to the hub of the introducer sheath, the dilator clip comprising a base having a distal portion and a dilator disposed on the distal portion of the base, wherein the dilator is coaxially positioned within the lumen of the introducer sheath.

Preferably, the dilator is integral with the dilator clip.

In an embodiment, the introducer assembly includes an introducer sheath having a proximal end. A lumen is disposed longitudinally through the interior of the introducer sheath. A hub is attached about the proximal end of the sheath. The hub includes an opening disposed through its center. The opening is in fluid communication with the lumen of the introducer sheath. A dilator clip is releasably attached to the hub of the introducer sheath. The dilator clip comprises a base having a proximal portion and a distal portion. Disposed on the distal portion of the dilator clip is a dilator. The dilator is coaxially positioned within the lumen of the introducer sheath while the dilator clip is attached to the hub.

For one example, the one or more attachment regions may comprise a plurality of apertures configured to receive sutures. In other embodiments, the dilator clip includes engaging members configured to interlock with receiving members of the hub. The engaging members may include one or more clasps that engage the receiving members of the hub. In another embodiment, the dilator clip includes one or more flanges extending proximally from the base of the dilator clip to manipulate the dilator. The flanges may have an ergonomic profile and may include ergonomic features such as nubs, grooves, and the like.

In another embodiment, a hub for an introducer assembly includes a main body. At least one opening is disposed through the main body. The hub further includes one or more receiving members disposed about the periphery of the main body. The one or more receiving members are configured to receive a dilator clip. In one embodiment, the hub has a concave configuration. In another example, the hub further includes a wire guide receiving member. The wire guide receiving member may include a plurality of hooks to secure the wire guide during the procedure.

In another embodiment, an introducer assembly includes an introducer sheath having a proximal end. A lumen is disposed longitudinally through the interior of the introducer sheath. A hub is attached about the proximal end of the sheath. The hub includes a main body having at least one opening disposed through its center in fluid communication with the lumen of the introducer sheath. The hub further includes a wire guide receiving member attached about a periphery of the main body. A dilator clip is releasably attached to the hub of the introducer sheath. The dilator clip comprises a base having a proximal portion and a distal portion. Disposed on the distal portion of the dilator clip is a dilator. The dilator is coaxially positioned within the lumen of the introducer sheath while the dilator clip is attached to the hub. In some embodiments, the dilator is integral with the dilator clip.

Other systems, features and advantages of the invention will be, or will become, apparent to one with skill in the art upon examination of the following figures and detailed description. It is intended that all such additional systems, methods, features and advantages be within the scope of the invention, and be encompassed by the following claims.

### BRIEF DESCRIPTION OF THE DRAWINGS

Embodiments of the present invention are described below, by way of example only, with reference to the accompanying drawings, in which:
FIG. 1 shows an embodiment of an introducer assembly having a hub and a dilator disposed therethrough;
FIG. 2 shows a perspective view of an embodiment of the hub of FIG. 1;
FIG. 3 shows a perspective view of an embodiment of a dilator clip for use with the introducer assembly of FIG. 1;
FIG. 4 shows a perspective view of the dilator clip engaged with the hub of FIG. 1;
FIGS. 5A and 5B show exemplary embodiments of a hub for an introducer assembly, not forming part of the current invention;
FIG. 6 shows a perspective view of the dilator clip engaged with the hub of FIG. 5B;
FIG. 7 shows another exemplary embodiment of a hub for an introducer assembly;

### DETAILED DESCRIPTION OF THE PREFERRED EMBODIMENTS

Unless defined otherwise, all technical and scientific terms used herein have the same meaning as commonly understood by one of ordinary skill in the art to which this invention pertains.

The term "prosthesis" means any device for insertion or implantation into, or replacement, for a body part or function of that body part. It may also mean a device that enhances or adds functionality to a physiological system. The term prosthesis may include, for example and without limitation, a stent, stent-graft, filter, valve, balloon, embolization coil, and the like.

The term "endoluminal" refers to or describes the internal or inside of a lumen, duct, and other passageways or cavities located in a human or other animal body. A lumen or a body passageway may be an existing lumen or a lumen created by surgical intervention. As used in this specification, the terms "lumen" or "body passageway," and "vessel" are intended to have a broad meaning and encompass any duct (e.g., natural or iatrogenic) or cavity within the human body and may include, without limitation, blood vessels, respiratory ducts, gastrointestinal ducts, such as the biliary duct, intestines, the esophagus, the pericardial cavity, the thoracic cavity, the pericardial cavity, and the like. Accordingly, the terms "endoluminal device" or "endoluminal prosthesis" describe devices that can be placed inside or moved through any such lumen or duct.

The terms "patient," "subject," and "recipient" as used in this application may refer to any animal, particularly humans.

The term "biocompatible" refers to a material that is substantially non-toxic in the in vivo environment of its intended use, and that is not substantially rejected by the patient's physiological system (i.e., is non-antigenic). This can be gauged by the ability of a material to pass the biocompatibility tests set forth in International Standards Organization (ISO) Standard No. 10993 and/or the U.S. Pharmacopeia (USP) 23 and/or the U.S. Food and Drug Administration (FDA) blue book memorandum No. G95-1, entitled "Use of International Standard ISO-10993, Biological Evaluation of Medical Devices Part-1: Evaluation and Testing." Typically, these tests measure a material's toxicity, infectivity, pyrogenicity, irritation potential, reactivity, hemolytic activity, carcinogenicity and/or immunogenicity. A biocompatible structure or material, when introduced into a majority of patients, will not cause a significantly adverse, long-lived or escalating biological reaction or response, and is distinguished from a mild, transient inflammation which typically accompanies surgery or implantation of foreign objects into a living organism.

The term "medical device" means any object that is itself or that includes a component that is intentionally inserted into the body of a patient as part of a medical treatment, and that comprises a structure adapted for introduction into a patient. The medical device can be a tool, such as, without limitation, a catheter, a wire guide, a forceps, or a scissors used to affect a surgical procedure at and/or deliver a second medical device to a treatment site in a patient.

In the following discussion, the terms "proximal" and "distal" will be used to describe the opposing axial ends of the assembly, as well as the axial ends of various components thereof. The term "proximal" is used in its conventional sense to refer to the end of the assembly (or component thereof) that is the closest to the operator during use of the assembly. The term "distal" is used in its conventional sense to refer to the end of the assembly (or component thereof) that is initially inserted into the patient, or that is closest to the patient during use.

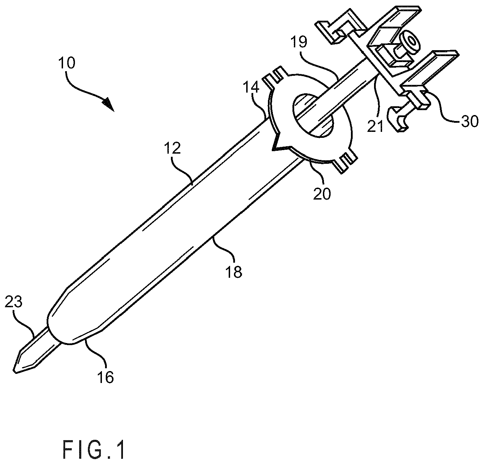

FIGS. 1-3 generally show an embodiment of illustrative introducer assembly 10 for performing a percutaneous nephrostomy. The introducer assembly 10 includes an introducer sheath 12 having a proximal end 14. A lumen is disposed longitudinally through the interior of the introducer sheath 12. A hub 20 is attached about the proximal end 14 of the introducer sheath 12. The hub 20 includes an opening disposed through its center. The opening is in fluid communication with the lumen of the introducer sheath. A dilator clip 30 is releasably attached to the hub 20 of the introducer sheath 12. The dilator clip 30 comprises a base 32 having a proximal portion 31 and a distal portion 33. Disposed on the distal portion 33 of the dilator clip 30 is a dilator 19. The dilator 19 is coaxially positioned within the lumen of the introducer sheath 12 while the dilator clip 30 is attached to the hub 20.

In some embodiments, the introducer assembly 10 is intended to deliver endoluminal devices, such as a ureteral stent, into a patient through a percutaneous nephrostomy procedure in which the introducer assembly 10 is advanced through a person's skin to reach the calices of the kidney directly. However, the introducer assembly 10 is not limited for use in a percutaneous nephrostomy procedure but may be used in other procedures. For instance, if a path to a bile duct is needed, the physician may access the bile duct through an endoscope via the mouth, esophagus, stomach and intestines, or via laparoscopic methods directly through the skin (percutaneous). If vascular access is desired, a physician may access the blood vessel through an opening, such as an opening manufactured in the femoral artery.

FIG. 1 shows a perspective view of an introductory assembly 10. As shown, the introducer assembly 10 includes an introducer sheath 12, hub 20, and a dilator clip 30. The introducer sheath 12 has an elongated tubular body having a wall thickness and a proximal end 14 and a tapered distal end 16. The introducer sheath 12 is designed to be inserted through an orifice of the patient and run through a passage way to reach a specific area of the patient for a surgical procedure. In one particular embodiment, the introducer sheath 12 may be formed as a composite of two or more layers of the same or different materials that are laminate or otherwise mechanically bonded together. An inner surface of the introducer sheath 12 defines a lumen extending along a longitudinal access thereof. The lumen may have a generally constant diameter along its length. The introducer sheath 12 may have a uniform internal diameter.

The introducer sheath 12 may be made from any suitable biocompatible material, for example polytetrafluoroethylene (PTFE), nylon, or polyethylene. In this embodiment, the introductory sheath 12 includes an outer layer 18. The outer layer 18 may comprise a low friction non rigid or semi rigid material. The outer layer 18 of the introducer sheath 12 may include one or more discrete PEBAX sections that vary in durometer from a high durometer at the proximal end 14 of the sheath 12 to a lower durometer at the distal end 16 of the sheath 12. The introducer sheath 12 may optionally include a flat wire coil or braid disposed between or incorporated into the one or more layers to the introducer sheath 12 with increased pushability and kink-resistance as the introducer sheath 12 is introduced into the body lumen or cavity, as described in U.S. Patent Numbers 5,380,304 and 5,700,253. To increase lubricity, the outer layer 18 of the introducer sheath 12 may be coated with a hydrophilic coating.

The introducer sheath 12 further includes a hub 20 positioned at its proximal end. The hub 20 is used to maintain the introducer sheath 12 in the desired position upon the outer skin surface of the patient. The hub 20 may be comprised from materials having a high flex modulus such as ABS plastic and nylon. The hub 20 may be manufactured using any suitable process conventionally used to shape polymeric materials such as thermoplastic and elastomeric materials. Examples of such shaping processes include, but are not limited to, injection molding, insert molding, calendaring, and casting.

A dilator 19 may be introduced simultaneously, or jointly, with the introduction of the introducer sheath. The dilator 19 is used by the physician to dilate a tract, or passage, for the introducer sheath 12. The dilator 19 has a tubular shaft body having a proximal end 21 and a distal end 23. A lumen (not shown) may be disposed completely therethrough from the proximal end 21 to the distal end 23. The dilator 19 is generally sized to fit within the inner diameter of the sheath 12, and has a length greater than the length of the sheath 12. Accordingly, the outer diameter of the dilator 19 should be sized to be smaller than the inner diameter of the introducer sheath 12. The size of the dilator 19 may range from about 2 mm (6 Fr) to about 10 mm (30 Fr).

As shown in the embodiment of FIG. 1, the dilator 19 is attached to a dilator clip 30. As will be discussed in further detail below, the dilator clip 30 may be releaseably attached to the hub 20 of the introducer assembly 10 to further facilitate simultaneous introduction of the dilator 19 and the introducer sheath 12. In some embodiments, the dilator 19 may be molded onto the dilator clip 30. In other embodiments, the dilator 19 may be attached to the dilator clip 30 by other known means, including by adhesives. In other embodiments the dilator 19 is integrally formed with the dilator clip 30. The dilator 19 can be made from biocompatible materials such as polyethylene, polyamide, polyurethane or vinyl, or any combination of these materials. One particular preferred composition is a lubricous fluropolymer composition, such as PTFE. The dilator clip 30 may be manufactured using any suitable process conventionally used to shape polymeric materials such as thermoplastic and elastomeric materials. Among such shaping processes are included, but not limited to injection molding, insert molding, calendaring, and casting.

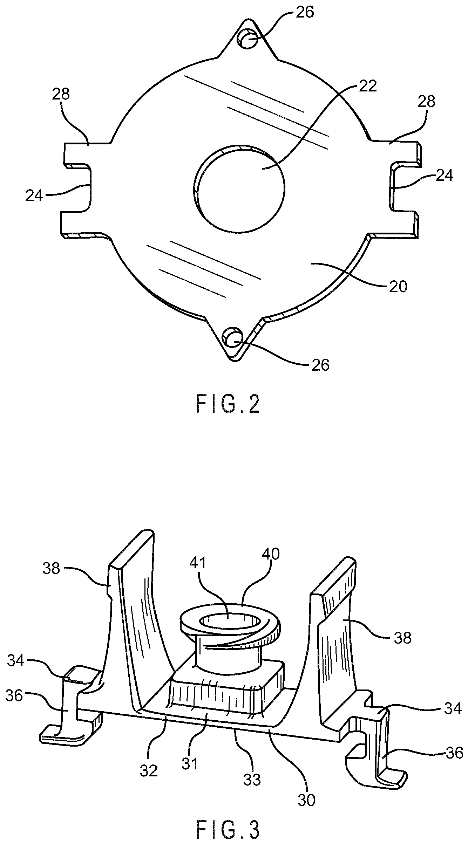

FIG. 2 shows the hub 20 in greater detail. As shown, the hub 20 is in the form of a generally circular disc. The hub 20 includes an opening 22 disposed through the center of the hub 20, a pair of receiving members 24, and a plurality of attachment regions 26. The opening 22 is configured to receive a portion of the introducer sheath 12. The diameter of the opening 22 may vary depending on the outer diameter of the outer layer 18 of the introducer sheath 12 required to perform the endoluminal procedure. The diameter of the opening 22 is sized to be substantially the same as the outer diameter of the outer layer 18 of the introducer sheath 12. In particular, the diameter of the opening 22 may range from 6 mm to 10 mm (18 Fr to 30 Fr). The hub 20 may further include an extension (not shown) disposed about the periphery of the opening 22 designed to help facilitate the attachment of the outer layer 18 of the introducer sheath 12 to the hub 20. The introducer sheath 12 may be fixedly secured to the hub 20 by methods including, but not limited to, insert molding, adhesives, or via any other methods known in the art. Preferably, the hub 20 is molded to the outer layer 18 of the introducer sheath 12.

The pair of are configured to engage with the portions of a dilator clip 30, as will be discussed below. As shown in this embodiment, the receiving members comprise two recessed areas positioned on opposite sides of the hub 20. Each receiving member 24 has generally a "U-Shape," which provides a recess formed between a pair of walls 28 to receive the corresponding engaging members of the dilator clip 30. The distance between the walls 28 may be about 0.47 cm. Other embodiments of the hub 20 may comprise receiving members having different configurations. In one embodiment, the receiving members 24 may include regions configured to engage with corresponding teeth on the dilator clip 30. The hub 20 also includes attachment regions 26 disposed about the periphery of the hub 20. The attachment regions 26 are positioned on opposite sides of the hub 20 that do not include a pair of receiving members 24. The attachment regions 26 may comprise a pair of apertures, which may receive sutures or other devices in order to secure the hub 20 to the skin of the patient. The attachment regions 26 may have a diameter of about 0.20 cm. In other embodiments, the hub 20 may be secured to the skin surface of a patient through the use of biocompatible adhesives or other connecting devices.

FIG. 3 shows a perspective view of the dilator clip 30 used with the introducer assembly 10. The dilator clip 30 comprises a base 32, a pair of engaging members 34 positioned on opposing sides of the dilator clip 30, and a pair of flanges 38. The base 32 has a generally rectangular configuration. In other embodiments, the base 32 may comprise other configurations, including, but not limited to, circular or elliptical. The base 32 includes a proximal portion 31 and a distal portion 33. The dilator clip 30 includes engaging members 34 attached to the distal portion of the base 32. As discussed with regard to the hub 20 of the introducer sheath 12, the engaging members 34 of the dilator clip 30 are designed to interlock with the receiving members 24 of the hub 20. In this embodiment, the engaging members 34 comprise a pair of clasps 36 extending in a distal direction from the base 32. The clasps 36 are positioned on opposing sides of the base 32. Other configurations for engaging members may be used with the dilator clip 30. Each clasp 36 may range in size from about 0.15 cm to about 0.38 cm. Preferably, each clasp 36 has a size of 0.25 cm.

The engaging members 34 are manipulated by the pair of flanges 38 attached to the proximal portion 31 of the base 32 and extending in a proximal direction from the base 32 of the dilator clip 30. The flanges 38 may be formed integrally with the base 32 of the dilator clip 30 or may be separately attached to the base 32. In a preferred embodiment, the flanges 38 are insert molded during the manufacturing process of the dilator clip 30. The flanges 38 may be provided with an ergonomic grip shape. The ergonomic shape allows a physician to conveniently grasp and manipulate both the dilator 19 and the introducer sheath 12 during introduction of both of those devices when the dilator clip 30 is attached to the hub 20. In addition to the ergonomic profile, the flanges 38 may also include additional ergonomic surfaces, such as nubs, grooves, and the like, disposed about the exterior of the flanges 38. The flanges 38 of the dilator clip 30 may have a length of about 0.58 cm. Other embodiments of the dilator clip 30 may include flanges 38 having lengths greater than 0.58 cm in order to accommodate the user of the dilator clip 30. The dilator clip 30 may have an associated connecting adaptor 40 disposed on the proximal portion 31 of the base 32. The connecting adaptor 40 has a central opening 41 that is in fluid communication with the lumen of the dilator 19. The connecting adaptor 40 preferably has a male Luer lock thread to facilitate the attachment of tubing or a syringe to the dilator clip 30.

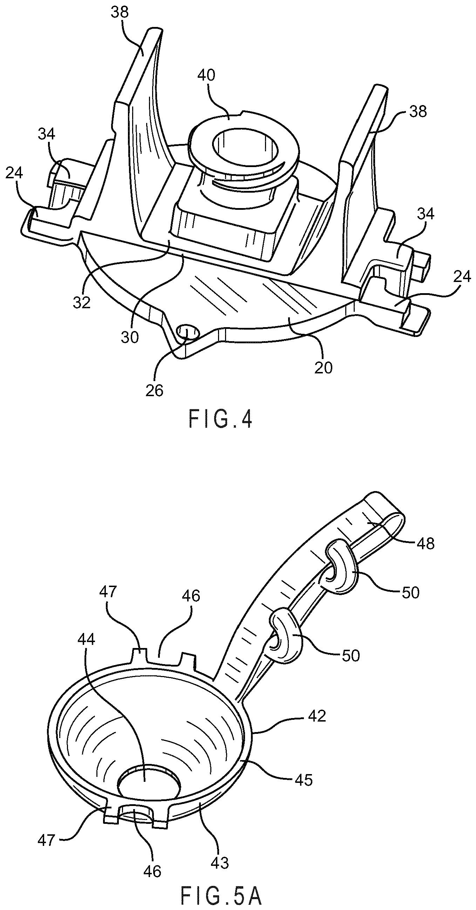

FIG. 4 illustrates the interaction between the dilator clip 30 and the hub 20. As shown, the engaging members 34 of the dilator clip 30 interlock with the receiving members 24 of the hub 20 to form a snap-fit. The receiving members 24 of the hub 20 and the engaging members 34 of the dilator clip 30 are designed to provide a releasable attachment between the dilator clip 30 and the hub 20 of the introducer assembly 10. When the dilator clip 30 is attached to the hub 20 of the introducer sheath 12, both the dilator 19 and the introducer sheath 12 can be introduced into the body of the patient in a single step. This improvement allows a physician to minimize trauma to the patient due to the introduction of the medical devices separately. The releasable attachment of the dilator clip 30 to the hub 20 also allows the physician to remove the dilator 19 while the introducer sheath 20 maintains the tract dilated by the dilator 19.

An exemplary method of using the introducer assembly 10 is described below. Prior to performing the procedure, the physician introduces an interventional device, such as a cannula and stylet, into the body of the patient to create an access tract. A guide wire is then inserted into the body opening over, or through, the cannula and stylet. Following the insertion of the access tract, a guide wire is introduced into the patient. With the dilator clip 30 attached to the hub 20 of the introducer sheath 12, the physician introduces the introducer sheath 12 and the dilator 19 simultaneously over the guide wire. The dilator 19 is used to expand the tract to accommodate the introducer sheath 12. As the dilator 19 is advanced into the body, the access tract gradually increases in diameter as a result of the shape of the distal end 23 of the dilator 19. The hub 20 is secured to the outer skin surface of the patient by placing sutures through the attachment regions 26 of the hub 20. Following the expansion of the tract, the physician may remove the dilator 19 from the body of the patient by utilizing the flanges 38 on the dilator clip 30 to release the engaging members 34 from the receiving members of the hub 20. The physician may then introduce a prosthesis, such as a stent, into the body of the patient through the introducer sheath 12. After the prosthesis is implanted, the physician can detach the hub from the patient's skin and retract the introducer sheath 12.

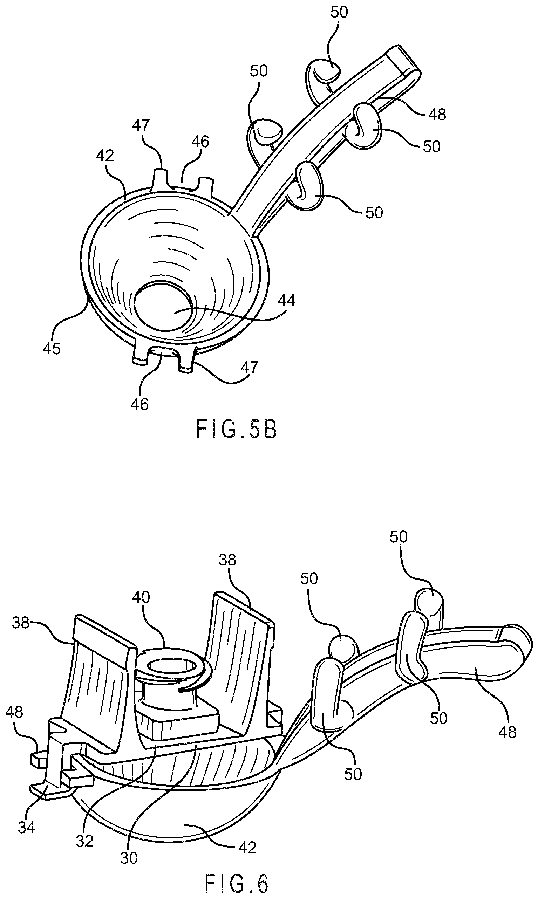

FIGS. 5A and 5B show exemplary embodiments of a hub 42 for use with an introducer assembly, not forming part of the current invention. The hub 42 comprises a main body 43, a pair of receiving members 46, and a wire guide receiving members 48. As shown, the main body 43 has a hemispherical, or bowl-like, configuration. The main body 43 of the hub 42 includes a first opening 44 defining a first perimeter configured to receive a portion of the introducer sheath 12. The diameter of the first opening 44 may vary depending on the outer diameter of the introducer sheath 12 required to perform the particular procedure by the physician. The diameter of the first opening 44 is sized to be substantially the same as the outer diameter of the outer layer of the introducer sheath 12. In particular, the diameter of the first opening 44 may range from 6mm to 10 mm (18 Fr to 30 Fr). The main body 43 of the hub 42 also includes a second opening 45 defining a second perimeter. The diameter of the second opening 45 is greater than the diameter of the first opening 44. In particular, the second opening 45 may have a diameter in the range of about 1.00 cm to about 4.00 cm. In an feature of the embodiment shown in FIGS. 5A and 5B, the second opening may have a diameter of about 2.79 cm. The diameter of the main body 43 increases throughout its depth in a proximal direction. The main body 43 may have a depth in the range of about 0.75 cm to about 2.00 cm. In one embodiment the main body may have a depth of about 1.14 cm.

The hub 42 further includes a pair of receiving members 50. The receiving members may comprise a plurality of configurations. In the embodiments shown in FIGS. 5A and 5B, the receiving members 46 comprise two oppositely positioned recessed areas. The receiving members 46 are configured to engage with the engaging members 34 of the dilator clip 30, as discussed above. The distance between walls 47 of the recessed area may range from about 0.25 cm to about 0.75 cm. In one feature of the embodiments shown in FIGS. 5A and 5B, the distance between the walls 47 of the recessed areas may be about 0.47 cm. The hub 42 also includes a wire guide receiving member 48. The wire guide receiving member 48 is attached to the main body 43 of the hub 42 and extends proximally from the main body 43 of the hub 42. The wire guide receiving member 48 may include one or more hooks 50 for retaining a wire guide. In the embodiment shown in FIG. 5A, a pair of hooks 50 is disposed on a single side of the wire guide receiving member 48 configured to engage and retain a wire guide during the procedure. In some embodiments, as shown in FIG. 5B, each side of the wire guide receiving member 48 includes a pair of hooks 50 configured to engage and retain a wire guide during the procedure. The length and width of the wire guide receiving member 48 may be determined based on the size of the wire guide necessary to perform the desired procedure. For example, the length and width of the wire guide receiving member 48 may be increased to facilitate a wire guide having a large diameter. In one embodiment, the wire guide receiving member 48 may have a general length of about 2.54 cm and a width of about 0.64 cm. The hooks disposed on the wire guide receiving member 48 may each have a thickness of about 0.32 cm.

FIG. 6 illustrates the interaction between the dilator clip 30 and the hub 42. As shown, the engaging members of the dilator clip 30 interlock with the receiving members 46 of the hub 42 to form a snap-fit. The receiving members 46 of the hub 42 and the engaging members 34 of the dilator clip 30 are designed to provide a releasable attachment between the dilator clip 30 and the hub 42 of the introducer sheath 12.

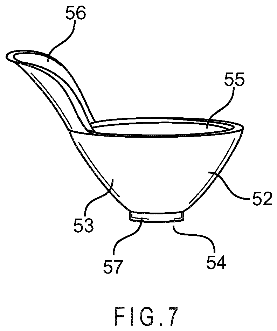

FIG. 7 shows another exemplary embodiment of the hub 52 for an introducer assembly, not forming pat of the current invention. The hub 52 comprises a main body 53 and a wire guide receiving member 56. As shown, the main body 53 has a tapered, or conical, configuration. The hub 52 includes a first opening 54 defining a first perimeter configured to receive a portion of an introducer sheath. The diameter of the first opening 54 may vary depending on the outer diameter of the introducer sheath required to perform the particular procedure by the physician. The diameter of the first opening 54 is sized to be substantially the same as the outer diameter of the outer layer of the introducer sheath. The main body 53 of the hub 52 also includes a second opening 55 defining a second perimeter. The diameter of the second opening 55 is greater than the diameter of the first perimeter. In particular, the second opening 55 may have a diameter in the range of about 1.00 cm to about 4.00 cm. In an feature of the embodiment shown in FIG. 7, the second opening 55 may have a diameter of about 2.16 cm. The hub 52 may have a depth of about 1.14 cm. The diameter of the main body 53 increases throughout the depth of the main body 53 in proximal direction. The wire guide receiving member 56 is attached to the main body 53 of the hub 52 and extends proximally from the main body of the hub 52. The wire guide receiving member 56 may have a general length of about 0.450 cm and a width of about 0.64 cm. The hub 52 may include pair of receiving members configured to engage with the engaging members of a dilator clip 30, as discussed above. The hub 52 further includes an extension 57 disposed about the periphery of the first opening 54 designed to help facilitate the attachment of the outer layer of an introducer sheath to the hub 52.

Throughout this specification various indications have been given as to preferred and alternative embodiments of the invention. However, the foregoing detailed description is to be regarded as illustrative rather than limiting and the invention is not limited to any one of the provided embodiments. It should be understood that it is the appended claims, including all equivalents, that are intended to define the scope of this invention.

All optional and preferred features and modifications of the described embodiments forming part of the invention and dependent claims are usable in all aspects of the invention taught herein. Furthermore, the individual features of the dependent claims, as well as all optional and preferred features and modifications of the described embodiments are combinable and interchangeable with one another.

## Claims

1. An introducer assembly (10), including:
an introducer sheath (12) having a proximal end and a lumen disposed therethrough,
a hub (20) attached about the proximal end of the introducer sheath, the hub having an opening (22) disposed through its center in fluid communication with the lumen of the introducer sheath, the hub comprising a pair of receiving members (24) positioned about the periphery of the hub on opposite sides, each receiving member having a recess formed between a pair of walls (28), and one or more attachment regions (26) for securing the hub to the skin surface positioned on opposite sides of the hub that do not include the pair of receiving members;
and a dilator clip (30) releasably attached via the receiving members (24) to the hub of the introducer sheath, the dilator clip including a base (32) having a proximal portion (31) and a distal portion (33) and a dilator (19) disposed on the distal portion of the base,
wherein the dilator is coaxially positioned within the lumen of the introducer sheath.

2. An introducer assembly according to claim 1, wherein the diameter of the opening (22) of the hub ranges from about 6 mm to about 10 mm (about 18 Fr to about 30 Fr).

3. An introducer assembly according to claim 1 or 2, where the hub (20) has a circular or a concave configuration.

4. An introducer assembly according to any preceding claim, wherein the dilator clip (30) includes engaging members (34) configured to interconnect with the receiving members (24) of the hub.

5. An introducer assembly according to claim 4, wherein the engaging members are clasps (36).

6. An introducer assembly according to any preceding claim, wherein the dilator clip (30) includes:
one or more flanges (38) extending proximally from the base of the dilator clip; and/or
a connector (40) disposed on the base.

7. An introducer assembly according to claims 4 or 5, and claim 6, wherein the engaging members (34) are manipulated by the flanges (38) attached to the proximal portion of the base.

8. An introducer assembly according to any preceding claim, wherein the base (32) of the dilator clip has a generally rectangular shape.

9. An introducer assembly according to any of claims 4 to 8, wherein the engaging members (34) are positioned on opposing sides of the dilator clip.

10. An introducer assembly according to any preceding claim, wherein the hub (20) is a circular disc.

11. An introducer assembly according to any of claims 4 to 10, wherein the engaging members (34) interlock with the receiving members (24) to form a snap-fit.

## Patentansprüche

1. Einführvorrichtung (10), umfassend:
eine Einführhülle (12) mit einem proximalen Ende und einem hindurchgehend angeordneten Lumen,
eine Nabe (20), die um das proximale Ende der Einführhülle angeordnet ist, wobei die Nabe eine Öffnung (22) durch ihre Mitte in Fluidverbindung mit dem Lumen der Einführhülle angeordnet aufweist, wobei die Nabe ein Paar von Aufnahmeelementen (24) umfasst, die um den Rand der Nabe an gegenüberliegenden Seiten angeordnet sind, wobei jedes Aufnahmeelement eine Vertiefung, die zwischen einem Paar von Wänden (28) gebildet ist, aufweist, und einen oder mehrere Befestigungsbereiche (26) zum Befestigen der Nabe an der Hautoberfläche, die an gegenüberliegenden Seiten der der Nabe angeordnet sind, die das Paar von Aufnahmeelementen nicht umfassen;
und eine Dilatorklemme (30), die über die Aufnahmeelemente (24) lösbar an der Nabe der Einführhülle befestigt ist, wobei die Dilatorklemme eine Basis (32) mit einem proximalen Teil (31) und einem distalen Teil (33) und einen an dem distalen Teil des Basis angeordneten Dilator (19) aufweist,
wobei der Dilator koaxial in dem Lumen der Einführhülle angeordnet ist.

2. Einführvorrichtung gemäß Anspruch 1, wobei der Durchmesser der Öffnung (22) der Nabe in dem Bereich von etwa 6 mm bis etwa 10 mm (etwa 18 Fr bis etwa 30 Fr) liegt.

3. Einführvorrichtung gemäß Anspruch 1 oder 2, wobei die Nabe (20) eine kreisförmige oder eine konkave Konfiguration aufweist.

4. Einführvorrichtung gemäß einem der vorstehenden Ansprüche, wobei die Dilatorklemme (30) Eingriffselemente (34) aufweist, die zur Verbindung mit den Aufnahmeelementen (24) der Nabe gestaltet sind.

5. Einführvorrichtung gemäß Anspruch 4, wobei die Eingriffselemente Klammern (36) sind.

6. Einführvorrichtung gemäß einem der vorstehenden Ansprüche, wobei die Dilatorklemme (30) umfasst:
eine oder mehrere Laschen (38), die proximal von der Basis der Dilatorklemme vorragen; und/oder
ein Verbindungselement (40), das an der Basis angeordnet ist.

7. Einführvorrichtung gemäß Ansprüchen 4 oder 5 und Anspruch 6, wobei die Eingriffselemente (34) über die Laschen (38), die an dem proximalen Teil der Basis angebracht sind, manipuliert werden.

8. Einführvorrichtung gemäß einem der vorstehenden Ansprüche, wobei die Basis (32) der Dilartorklemme eine allgemein rechteckige Form aufweist.

9. Einführvorrichtung gemäß einem der Ansprüche 4 bis 8, wobei die Eingriffselemente (34) an gegenüberliegenden Seiten der Dilatorklemme angeordnet sind.

10. Einführvorrichtung gemäß einem der vorstehenden Ansprüche, wobei die Nabe (20) eine kreisförmige Scheibe ist.

11. Einführvorrichtung gemäß einem der Ansprüche 4 bis 10, wobei die Eingriffselemente (34) mit den Aufnahmeelemente (24) verriegeln, um einen Schnappverschluss zu bilden.

## Revendications

1. Ensemble introducteur (10), comportant :
une gaine (12) d'introducteur possédant une extrémité proximale et une lumière disposée à travers celle-ci,
un embout (20) fixé autour de l'extrémité proximale de la gaine d'introducteur, l'embout possédant une ouverture (22) disposée à travers son centre en communication fluidique avec la lumière de la gaine d'introducteur, l'embout comprenant une paire d'éléments de réception (24) positionnés autour de la périphérie de l'embout sur des côtés opposés, chaque élément de réception possédant un renfoncement formé entre deux parois (28), et une ou plusieurs régions de fixation (26) permettant de fixer l'embout à la surface cutanée positionnée sur des côtés opposés de l'embout qui ne comprennent pas les deux éléments de réception ;
et une attache (30) de dilatateur fixée de manière amovible par l'intermédiaire des éléments de réception (24) à l'embout de la gaine d'introducteur, l'attache de dilatateur comprenant une base (32) possédant une partie proximale (31) et une partie distale (33) et un dilatateur (19) disposé sur la partie distale de la base,
dans lequel le dilatateur est positionné coaxialement à l'intérieur de la lumière de la gaine d'introducteur.

2. Ensemble introducteur selon la revendication 1, dans lequel le diamètre de l'ouverture (22) de l'embout se situe dans la plage d'environ 6 mm à environ 10 mm (environ 18 Fr à environ 30 Fr).

3. Ensemble introducteur selon la revendication 1 ou 2, dans lequel l'embout (20) présente une configuration circulaire ou concave.

4. Ensemble introducteur selon l'une quelconque des revendications précédentes, dans lequel l'attache (30) de dilatateur comprend des éléments de mise en prise (34) conçus pour une interconnexion avec les éléments de réception (24) de l'embout.

5. Ensemble introducteur selon la revendication 4, dans lequel les éléments de mise en prise sont des fermoirs (36).

6. Ensemble introducteur selon l'une quelconque des revendications précédentes, dans lequel l'attache (30) de dilatateur comprend :
une ou plusieurs brides (38) s'étendant proximalement depuis la base de l'attache de dilatateur ; et/ou
un raccord (40) disposé sur la base.

7. Ensemble introducteur selon les revendications 4 ou 5, et la revendication 6, dans lequel les éléments de mise en prise (34) sont manipulés par les brides (38) fixées à la partie proximale de la base.

8. Ensemble introducteur selon l'une quelconque des revendications précédentes, dans lequel la base (32) de l'attache de dilatateur a une forme généralement rectangulaire.

9. Ensemble introducteur selon l'une quelconque des revendications 4 à 8, dans lequel les éléments de mise en prise (34) sont positionnés sur des côtés opposés de l'attache de dilatateur.

10. Ensemble introducteur selon l'une quelconque des revendications précédentes, dans lequel l'embout (20) est un disque circulaire.

11. Ensemble introducteur selon l'une quelconque des revendications 4 à 10, dans lequel les éléments de mise en prise (34) s'interverrouillent avec les éléments de réception (24) afin de former un ajustement à encliqueter.
